# EUROPEAN PATENT SPECIFICATION

(11) **EP 3 067 416 B1**
(45) Date of publication and mention of the grant of the patent: **01.04.2020**
(21) Application number: 14860811.0
(22) Date of filing: 25.08.2014
(51) Int. Cl.: C12N 5/10, C12N 15/85, C12N 5/071, C12N 15/87, C12N 5/0786

(54) **METHOD FOR INTRODUCING EXOGENOUS MITOCHONDRIA INTO MAMMALIAN CELLS**
VERFAHREN ZUR EINFÜHRUNG VON EXOGEN MITOCHONDRIEN IN SÄUGETIERZELLEN
PROCÉDÉ POUR INTRODUIRE DES MITOCHONDRIES EXOGÈNES DANS DES CELLULES DE MAMMIFÈRE

(30) Priority: 08.11.2013 CN 201310554218
(43) Date of publication of application: 14.09.2016
(73) Proprietor: Guangzhou Institute Of Biomedicine And Health, Chinese Academy Of Sciences, Guangzhou, Guangdong 510530 (CN)
(72) Inventor: LIU, Xingguo, Guangzhou Guangdong 510530 (CN); PEI, Duanqing, Guangzhou Guangdong 510530 (CN); LIU, Jinglei, Guangzhou Guangdong 510530 (CN); LIU, Xuebin, Guangzhou Guangdong 510530 (CN); YAO, Deyang, Guangzhou Guangdong 510530 (CN)
(74) Representative: Appleyard Lees IP LLP
(86) International application number: PCT/CN2014/085117
(87) International publication number: WO 2015/067089

(56) References cited:
- WO-A2-03/012050
- WO-A2-2005/103229
- WO-A2-2007/038564
- WO-A2-2008/101233
- CN-A- 103 173 441
- CN-A- 103 205 463
- US-A1- 2013 149 778
- CLARK M A ET AL: "MITOCHONDRIAL TRANSFORMATION OF MAMMALIAN CELLS", NATURE, MACMILLAN JOURNALS LTD., ETC, vol. 295, no. 5850, 18 February 1982 (1982-02-18), pages 605-607, XP002625375, ISSN: 0028-0836
- EYAD KATRANGI ET AL: "Xenogenic Transfer of Isolated Murine Mitochondria into Human [rho] 0 Cells Can Improve Respiratory Function", REJUVENATION RESEARCH, vol. 10, no. 4, 1 December 2007 (2007-12-01), pages 561-570, XP055135802, ISSN: 1549-1684, DOI: 10.1089/rej.2007.0575
- DARIA MILESHINA ET AL: "Mitochondrial transfection for studying organellar DNA repair, genome maintenance and aging", MECHANISMS OF AGEING AND DEVELOPMENT, vol. 132, no. 8, 27 May 2011 (2011-05-27), pages 412-423, XP028298113, ISSN: 0047-6374, DOI: 10.1016/J.MAD.2011.05.002 [retrieved on 2011-05-27]
- COLLOMBET J M ET AL: "Introduction of plasmid DNA into isolated mitochondria by electroporation. A novel approach toward gene correction for mitochondrial disorders", JOURNAL OF BIOLOGICAL CHEMISTRY, AMERICAN SOCIETY FOR BIOCHEMISTRY AND MOLECULAR BIOLOGY, US, vol. 272, no. 8, 21 February 1997 (1997-02-21), pages 5342-5347, XP002221676, ISSN: 0021-9258, DOI: 10.1074/JBC.272.8.5342
- Y. G. YOON: "Transformation of isolated mammalian mitochondria by bacterial conjugation", NUCLEIC ACIDS RESEARCH, vol. 33, no. 16, 7 September 2005 (2005-09-07), pages e139-e139, XP055400098, ISSN: 0305-1048, DOI: 10.1093/nar/gni140
- DANIEL G GIBSON ET AL: "Enzymatic assembly of DNA molecules up to several hundred kilobases", NATURE METHODS, vol. 6, no. 5, 12 April 2009 (2009-04-12), pages 343-345, XP055224105, ISSN: 1548-7091, DOI: 10.1038/nmeth.1318
- TOMOYA KITANI ET AL: "Internalization of isolated functional mitochondria: involvement of macropinocytosis", JOURNAL OF CELLULAR AND MOLECULAR MEDICINE, 1 June 2014 (2014-06-01), pages n/a-n/a, XP055135790, ISSN: 1582-1838, DOI: 10.1111/jcmm.12316
- WEI, YUE ET AL.: 'Inheritance Analysis of Mitochondrial (mt)DNA in the Interspecific Crossing of Genus Cucumis' JOURNAL OF PLANT GENETIC RESOURCES vol. 12, no. 4, 31 August 2011, pages 612 - 618, XP055342812

### SEQUENCE LISTING

<110> GUANGZHOU INSTITUTES OF BIOMEDICINE AND HEALTH, CHINESE ACADEMY OF SCIENCES
<120> METHOD FOR INTRODUCING EXOGENOUS MITOCHONDRIA INTO A MAMMALIAN CELL
<130> P33248EPN1/SNB
<150> PCT/CN2014/085117
   <151> 2014-08-25
<150> CN201310554218.7
   <151> 2013-11-08
<160> 1
<170> Patent In version 3.5
<210> 1
   <211> 729
   <212> DNA
   <213> Artificial Sequence
<220>
   <223> GFP-COX-I fusion gene
<400> 1
   atggtgagca agggcgagga gctgttcacc ggggtggtgc ccatcctggt cgagctggac     60
   ggcgacgtaa acggccacaa gttcagcgtg tccggcgagg gcgagggcga tgccacctac    120
   ggcaagctga ccctgaagtt catctgcacc accggcaagc tgcccgtgcc ctggcccacc    180
   ctcgtgacca ccctgaccta cggcgtgcag tgcttcagcc gctaccccga ccacatgaag    240
   cagcacgact tcttcaagtc cgccatgccc gaaggctacg tccaggagcg caccatcttc    300
   ttcaaggacg acggcaacta caagacccgc gccgaggtga agttcgaggg cgacaccctg    360
   gtgaaccgca tcgagctgaa gggcatcgac ttcaaggagg acggcaacat cctggggcac    420
   aagctggagt acaactacaa cagccacaac gtctatatca tggccgacaa gcagaagaac    480
   ggcatcaagg tgaacttcaa gatccgccac aacatcgagg acggcagcgt gcagctcgcc    540
   gaccactacc agcagaacac ccccatcggc gacggccccg tgctgctgcc cgacaaccac    600
   tacctgagca cccagtccgc cctgagcaaa gaccccaacg agaagcgcga tcacatggtc    660
   ctgctggagt tcgtgaccgc cgccgggatc actctcggca tggacgagct gtacaaggcc    720
   gccgccacc                                                            729

## Description

### Technical Field

The present disclosure relates to the field of biological and genetic engineering, especially a method for introducing exogenous mitochondria into a mammalian cell.

### Background

Mitochondria are the most important organelles in eukaryotes, responsible for more than 90% of cellular energy supply. Mitochondria carry an independent genome, i.e., mitochondrial DNA, with independent gene transcription and protein translation configurations that are different from the nuclear genome. Mammalian mitochondrial DNA encodes 22 tRNAs, 2 rRNAs, and 13 polypeptides. These encoded polypeptides are critical subunits in a variety of protein complexes involved in aerobic respiration of mitochondria. A large number of studies have shown that mutations or reduced expression of these polypeptides can significantly inhibit cell aerobic respiration.

With the rapid development of modern molecular biology, people have been able to conduct a variety of genetic modification activities in the nuclear genome, such as gene knockin, gene knockout, site-directed mutagenesis, gene rearrangement, and so on. However, as of now, mitochondrial genetic modification technology is still very immature. People have only achieved mitochondrial genetic modification in yeast and other lower eukaryotes, but not yet in higher mammals. Many efforts have been reported regarding mitochondrial modification in mammals, but overall, none has been actually successful. One attempt was to express target genes in the nuclear genome, add localization sequences, and then introduce into mitochondria. There are problems with this approach. First of all, the majority of mammalian mitochondrial genome encodes functional RNAs, while the mechanism for RNAs encoded by nuclear genome to enter mitochondria has not been well understood. In a known mechanism, it is required to add a localization RNA sequence, yet it is unknown whether such a sequence would affect the functionality of target RNAs. On the other hand, after millions of years of evolution, the 13 polypeptides that currently remain in the mammalian mitochondrial genome differ from most nuclear genes. Expression of such polypeptides in nuclear genome may induce a problem of cytotoxicity. In addition, these polypeptides are often highly hydrophobic, which is the disadvantage of nuclear genome expression. Another attempt was to develop specific transfection media for mitochondria so as to transfect the target genes into mitochondria. Much has been reported in the literature in this aspect, but it is very difficult to transfer a target nucleic acid into mitochondria across its three-layer membrane structure from outside the cell. Few success is reported for methods using, for example, dequalinium plastids (DQAsome), polyethyleneimine (PEI) and derivatives. The methods reported also have few citations with poor reproducibility, and therefore cannot really achieve their goals. A third attempt was the use of techniques of nuclear transfer or cytoplasmic body hybridization, to increase the proportions of exogenous wild-type mitochondria in order to engineer the genetic behavior of mitochondria. Such methods cannot eradicate endogenous mitochondria and DNA, while it may additionally increase large amounts of exogenous cytoplasmic components and thus further increase uncertainty. Yet another attempt was to express TALEN molecules targeting mitochondria to disrupt the target sequence. This approach has been reported to successfully destroy mitochondrial DNA which has large deletions and specific point mutations. Being the most effective technical process for modifying mitochondrial DNA reported, this method is still insufficient in many ways. On one hand, for specific point mutations, it is highly doubted whether TALEN can specifically select a target sequence. On the other hand, this method can only achieve focused destruction of specific sequences, yet not site directed modifications or gene knockin. Mitochondria was discovered a hundred years ago, while mitochondrial DNA has been known for several decades. Efforts have been made aimed at mitochondrial DNA transformation ever since. However, until now, scientists have not been able to perform effective genetic modification of mammalian mitochondrial DNA. Effective mitochondrial genetic modification technique in mammalian cells thus remains a world-class technical problem.

The Craig J. Venter lab succeeded in obtaining synthetic whole sequences of mouse mitochondrial DNA with techniques in synthetic biology, but has not made any modifications thereto. Nor were they able to express the synthetic mitochondrial DNA in mammalian cells or identify the functionalities thereof.

### Summary of the Invention

Therefore, according to one aspect of the present disclosure, a macrophage comprising a synthetic mitochondrion is provided.

Specifically, the following technical solutions are disclosed.

The synthetic mitochondrion is obtained by introducing a synthetic mitochondrial DNA into a mitochondrion or an empty mitochondrial shell, the synthetic mitochondrial DNA is circular mitochondrial DNA containing a GFP-COX-I fusion gene, and is stably expressed and stably passaged in the macrophage after entering the macrophage.

Further aspects of the present disclosure are defined in the appended dependent claims.

According to another aspect of the present disclosure, a method for preparing a macrophage comprising the synthetic mitochondrion aforementioned is provided.

Specifically, the following technical solutions are disclosed.

The method for preparing a macrophage comprising a synthetic mitochondrion comprises adding the syntheticmitochondrion to a cell culture system comprising the macrophage and continuing culturing to obtain the macrophage comprising the synthetic mitochondrion.

The culturing conditions may be 36-38 °C in temperature and 4.5-5.5% CO₂ in a water-saturated closed incubator.

According to yet another aspect of the present disclosure, a synthetic mitochondrion is provided.

Specifically, the following technical solutions are disclosed.

A synthetic mitochondrion is provided, which may be obtained by introducing a synthetic mitochondrial DNA into a mitochondrion or an empty mitochondrial shell.

Method for preparing the synthetic mitochondrion aforementioned is also provided.

Accordingly, the method for preparing the synthetic mitochondrion may comprise:
obtaining a synthetic mitochondrial DNA;
preparing an empty mitochondrial shell, or obtaining a mitochondrion by isolation; and
introducing the synthetic mitochondrial DNA obtained from Step (1) into the empty mitochondrial shell or into the isolated mitochondrion obtained from Step (2) via eletroporation to obtain the synthetic mitochondrion.

The synthetic mitochondrial DNA, may be prepared by the following steps:
designing a new DNA sequence composition based on a mammalian mitochondrial DNA sequence using at least one gene modification means selected from the group consisting of gene introduction, gene knockout, site-directed mutagenesis, and gene rearrangement as needed, or, obtaining a wild-type DNA sequence composition; and
synthesizing DNA fragments of 50bp∼60bp according to the DNA sequence composition and connecting the DNA fragments synthesized with Gibson isothermal one-step method to obtain the synthetic mitochondrial DNA.

According to yet another aspect of the present disclosure, a method for introducing synthetic mitochondria into a macrophage is provided.

Specifically, the following technical solutions are disclosed.

Accordingly, the method for introducing synthetic mitochondria into a macrophage comprises:
(1) obtaining a synthetic mitochondrion by introducing a synthetic mitochondrial DNA into a mitochondrion or an empty mitochondrial shell, the synthetic mitochondrial DNA is circular mitochondrial DNA containing a GFP-COX-I fusion gene; and
(2) adding the synthetic mitochondrion obtained from Step (1) to a cell culture system comprising a macrophage and continuing culturing to obtain the macrophage comprising the synthetic mitochondrion, the synthetic mitochondrial DNA is stably expressed and stably passaged in the macrophage after entering the macrophage.

The synthetic mitochondrial DNA, may be prepared by the following steps:
(1) designing a new DNA sequence composition based on a mammalian mitochondrial DNA sequence using at least one gene modification means selected from the group consisting of gene introduction, gene knockout, site-directed mutagenesis, and gene rearrangement as needed, or, obtaining a wild-type DNA sequence composition; and
(2) synthesizing DNA fragments of 50bp∼60bp according to the DNA sequence composition and connecting the DNA fragments synthesized with Gibson isothermal one-step method to obtain the synthetic mitochondrial DNA.

In one of the embodiments, the culturing conditions may be 36-38 °C in temperature and 4.5-5.5% CO₂ in a water-saturated closed incubator.

The present disclosure utilizes synthetic biology to artificially synthesize a whole mitochondrial DNA sequence, realizing a fusion expression of GFP and COX-I based on a wild-type sequence, which provides a simple and effective means for molecular cloning of mitochondrial genome. We have successfully developed, for the first time, a method for introducing a synthetic mitochondrial DNA into a macrophage via endocytosis by a macrophage and obtained a synthetic mitochondrial DNA macrophage with stable GFP expression and stable passage.

The present disclosure is the first time in the world that molecular cloning operation has been successfully performed in mammalian mitochondrial DNA. The present disclosure introduces a synthetic mitochondrial DNA into a macrophage via endocytosis by macrophage, resulting in stable expression of genes from the synthetic mitochondrial DNA in macrophages. Such macrophages can be effectively passaged. Therefore, effective functionality of synthetic mitochondria in macrophages can be realized. The method for introducing a synthetic mitochondrial DNA into macrophages as disclosed herein may be used as a whole new mitochondrial molecular cloning means to perform site-directed mutagenesis, gene insertion, gene knockout, gene rearrangement, and the like in mitochondria. Therefore, any molecular cloning modification can be performed on a mammalian mitochondrial DNA with high purity and without any limitation in sources, which is of great importance to therapeutic schemes of diseases derived from mitochondrial DNA mutations.

### Brief Description of the Drawings

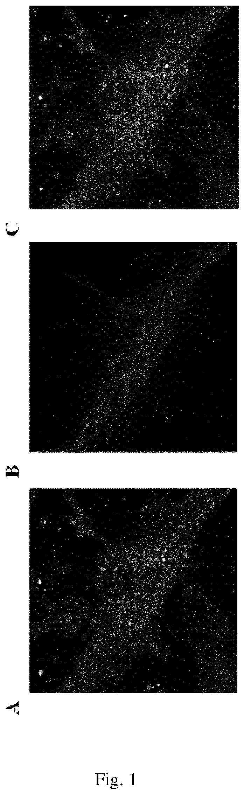

Figure 1 shows the result of exogenous mitochondria entering a macrophage as described in Example 1. 1A shows a DsRed2 detection result thereof, showing exogenous mitochondria originated from NIH3T3. 1B shows a corresponding EYFP detection result, showing endogenous mitochondria within the macrophage. 1c shows a result of two-color overlay.

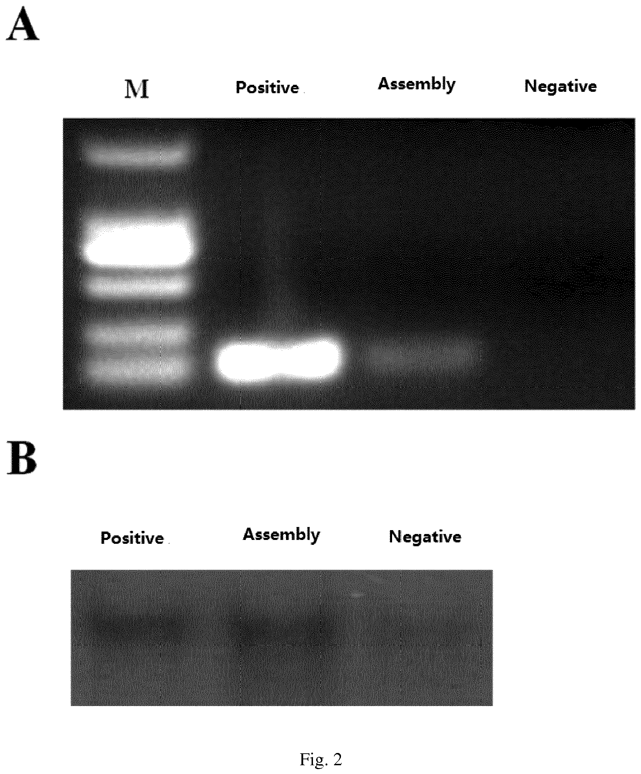

Figure 2 shows the *in vitro* assembly of synthetic mitochondria as described in Example 3. 2A shows the detection of properly transcript within the assembled synthetic mitochondria. 2B shows the detection of DNA replication within the assembled synthetic mitochondria. Description of symbols: M: molecular weight marker; Positive: positive control; Assembly: assembled synthetic mitochondria; Negative: negative control.

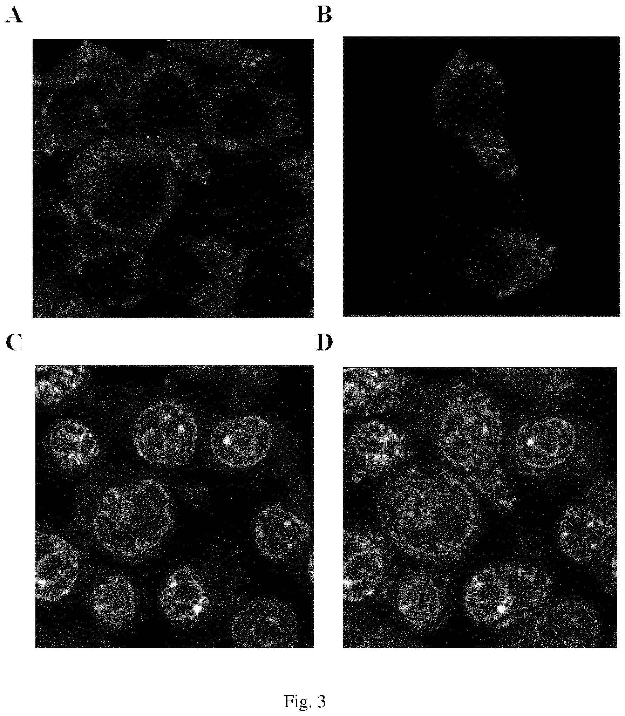

Figure 3 shows the fusion expression of GFP-COX I in mouse macrophage RAW264.7 as described in Example 4. 3A shows the locations of all mitochondria in the cell stained by Mito-Tracker Red dye. Mito-Tracker Red is a mitochondria-specific dye.3B shows the GFP signal of the green GFP-COX I fusion gene. 3C shows the DAPI staining results, indicating the location of the nucleus. 3D is a result of three-color overlay.

### Detailed Description of the Invention

The term "genetic modification" as used herein refers to construction of desoxyribonucleic acid (DNA) molecules of a target sequence via *in vitro* designing and total synthesis techniques. Genetic modifications as used herein involves designing a new DNA sequence, which may be exactly the same as a wild-type sequence or with any sequence modification as needed, including site-directed mutagenesis, gene introduction, gene knockout, and gene rearrangement.

Endocytosis, also known as endocytosis effect, is a process of transporting extracellular materials into a cell by the deformation movement of plasma membrane. Depending on the various size of engulfed substances and different endocytosis mechanisms, endocytosis can be divided into three types: phagocytosis, pinocytosis, and receptor-mediated endocytosis.

In order to facilitate clear understanding of the technical contents of the present disclosure, the following specific embodiments are described in details below with reference to the accompanying drawings. It should be understood that these embodiments are intended only for the purpose of illustrating the present invention, without any limitation to the scope thereof. Specific experimental methods that are not explicitly noted in the following examples are generally in accordance with conventional conditions, for example, as described in Sambrook et al., Molecular Cloning: A Laboratory Manual (New York: Cold Spring Harbor Laboratory Press, 1989), or according to the manufacturer's recommendations. All the chemical reagents involved in the embodiments are commercially available products.

### Example 1

This embodiment demonstrates that exogenous mitochondria may enter into mammalian cells by endocytosis. The endogenous mitochondria of the macrophages were labeled with EYFP by stably expressing fluorescent protein EYFP localized in mitochondria in macrophages. The endogenous mitochondria of the NIH3T3 cells were labeled with DsRed2 by stably expressing fluorescent protein DsRed2 localized in mitochondria in NIH3T3 cells. The NIH3T3 mitochondria labeled with DsRed2 were isolated and added to a macrophage culture system. Observations were made using a confocal microscope after 12 hours. It was observed that mitochondria labeled with DsRed2 entered macrophages and presented the same morphology as the endogenous mitochondria of the macrophages labeled with EYFP. Specifically:
1. Mouse macrophage cell line RAW264.7 cells and NIH3T3 cell culture medium (high glucose DMEM (commercially available from Hyclone, Item No: SH30022.01B); 10% fetal bovine serum (commercially available from GIBCO, Item No: 16000-044); double antibody (including 10000U / ml penicillin and 10000µg / ml streptomycin, 1: 100 dilution for use, available from GIBCO, Item No: 15140-122)). Cells were cultured in culture dishes according to conventional methods.
2. Plasmids mitoDsRed2 and mitoEYFP were constructed by means of molecular cloning. A mitochondrial localization sequence signal (MSVLTPLLLRGLTGSARRLPVPRAKIHSL) was attached to the N-terminus with standard DsRed2 (GenBank: AFS63392.1) and EYFP (GenBank: ACO48266.1) fluorescent protein sequences. The plasmids were constructed using pMXs vectors. Corresponding cells were transfected with the plasmids by electroporation and cultured via flow sorting monoclone culture to obtain cell lines stably expressing the fluorescent proteins.
3. The mitochondria were isolated with the use of Mitochondria/Cytosol Isolation Kit (APPLYGEN):
   (1) the cells were washed once with PBS, centrifuged at 6000rpm for 5min, and had the supernatant discarded;
   (2) cell pellet was collected from the tube bottom, re-suspended as cell suspension with 1.5ml Mito-Cyto buffer pre-cooled with ice, and mixed well, for example, via suction and discharge by 5ml syringe with extra small needle for about 40 times to yield cell homogenates;
   (3) the cell homogenate from above was transferred to a centrifuge tube and centrifuged at 800×g under 4°C for 5min, allowing the nucleus, large membrane fragments, unlysed cells, and the like to deposit to the bottom of the tube;
   (4) the pellet was discarded and the supernatant transferred to a new centrifuge tube, centrifuged again at 800×g under 4°C for 5min; and
   (5) the pellet was discarded and the supernatant transferred to a new centrifuge tube, centrifuged to 12,000×g under 4°C for 10min, with the empty mitochondria shells precipitated in the bottom of the tube.
4. The resultant precipitated mitochondrial shells from above were re-suspended with 200µL RAW264.7 cell culture medium. Aliquots of the suspension were added to culture dishes and incubated under 37°C and 5% CO₂ in a water-saturated closed incubator. Observations could be made in 12 hours after endocytosis, and the results are shown in Fig. 1.

### Example 2

In this embodiment, a circular mitochondrial DNA of designed sequence, i.e., a circular mitochondrial DNA containing GFP-COX-I fusion gene, is obtained by gene introduction, i.e., insertion, of GFP and Linker sequences, primer synthesis, and DNA splicing, to mouse mitochondrial DNA.

### Designing A new artificial circular mitochondrial DNA:

In this embodiment, a circular mitochondrial DNA is obtained by gene transfer, i.e., insertion, of GFP and Linker sequences, primer synthesis, and DNA splicing, to mouse mitochondrial DNA. Specifically, the sequence was derived from a known mitochondrial DNA sequences of wild type C57 BL / 6J mice (source of sequence: NCBI GenBank: EF108336) and GFP gene and Linker sequences were inserted in the position 5328 (specifically, as shown in Seq ID No.1), to obtain a designed circular mitochondrial DNA around 5Kb containing the GFP-COX-I fusion gene.
2. Depending on the specific composition of the designed circular mitochondrial DNA from above, conventional primer synthesis methods were used to obtain a plurality of DNA fragments to be spliced, which were around 50bp ∼ 60bp.
3. DNA splicing:
   (1) Based on the prior art, the Gibson isothermal one-step method was used for DNA splicing. The enzymatic systems employed in the Gibson isothermal one-step method were prepared as follows: mixing 320(µl 5X ISO buffer (25% PEG-8000,500mM Tris-HCl pH 7.5,50 mM MgCl2, 50 mM DTT, 4 dNTPs of 1 mM each, 5 mM NAD), 0.64µl 10 U/µl T5 exonuclease Epicentre, 20 µl 2 U/µl Phusion polymerase, and 160 µl 40 U/µl Taq ligase, filling with sterile water up to 1.2 ml, packing, and storing under -20 °C before use;
   (2) In the Gibson isothermal one-step method, the fragments to be spliced were mixed in equal proportions with a final concentration of 2 ng /µl. 5µl of the mixture were mixed evenly with15µl of the enzyme system described above and incubated at 50 °C for 1 hour to get properly spliced fragments.
4. The DNA was collected and purified. The spliced fragments were confirmed by sequencing to be the designed circular mitochondrial DNA above.

### Example 3

In this embodiment, the process of producing synthetic mitochondria through *in vitro* assembly of the circular mitochondrial DNA obtained in Example 2 and empty mitochondrial shells of NIH3T3 RhoO cells is described, as well as the extraction and identification processes of the RNA and DNA of the synthesized mitochondria. Figure 2 shows the detection of proper transcript (Fig. 2A) and DNA replicates (Fig. 2B) in the synthetic mitochondria.

### I. Culturing of the mitochondrion DNA-free RhoO free cells:

1. 1.5µg/mL ditercalinium or 250ng/mL ethidium bromide, 50µg/mL uridine (Sigma), and 110µg/mL sodium pyruvate were added to NIH3T3 cell culture medium;
2. Incubation was performed continuously for a month according to conventional methods;
3. NIH3T3 RhoO medium (high glucose DMEM, 10% fetal bovine serum, 50µg/mL uridine, 110µg/mL sodium pyruvate, double antibody of penicillin and streptomycin) was used for culturing;
4. Cells were collected, i.e., NIH3T3 RhoO cells without mitochondrial DNA were obtained.

### II. Isolation of empty RhoO mitochondrial shells:

The mitochondria were isolated with the use of Mitochondria/Cytosol Isolation Kit (APPLYGEN):
(1) the cells were washed once with PBS, centrifuged at 6000rpm for 5min, and had the supernatant discarded;
(2) cell pellet was collected from the tube bottom, re-suspended as cell suspension with 1.5ml Mito-Cyto buffer pre-cooled with ice, and mixed well, for example, via suction and discharge by 5ml syringe with extra small needle for about 40 times to yield cell homogenates;
(3) the cell homogenate from above was transferred to a centrifuge tube and centrifuged at 800×g under 4°C for 5min, allowing the nucleus, large membrane fragments, unlysed cells, and the like to deposit to the bottom of the tube;
(4) the pellet was discarded and the supernatant transferred to a new centrifuge tube, centrifuged again at 800×g under 4°C for 5min; and
(5) the pellet was discarded and the supernatant transferred to a new centrifuge tube, centrifuged to 12,000×g under 4°C for 10min, with the empty mitochondria shells precipitated in the bottom of the tube.

### III. Assembly of the mitochondrial shells and DNA sequences (electroporation):

(1) The empty mitochondrial shells were re-suspended with 50µl electroporation buffer (0.33M sucrose, 10% glycerol);
(2) 10µg mitochondrial genome (10µl TE buffer for the blank group) was added to the tube and mixed well;
(3) The well-mixed suspension was transferred into 1mm cuvette for an electric shock (parameters: field strength: 12 ∼ 16Kv/cm; capacitance: 25µF; and, resistance: 400Ω);
(4) After the shock, 1ml Incubation buffer 1 (40mM Tri-HCl 7.4; 25mM NaCl; 5mM MgCl2; 10% glycerol) was immediately added to the cuvette and mixed well with a pipette;
(5) The mixture was transferred to a new 2ml centrifuge tube and centrifuged at 21000g/min under 4°C for 10min, and the supernatant was discarded;
(6) The precipitation was washed twice using 1ml Incubation buffer, respectively.

### IV. RNA extraction, reverse transcription, and identification of the synthetic mitochondria:

1. Initial extraction of the synthetic mitochondrial RNA:
(1) The pellet was re-suspended with 50µl Incubation buffer 2 (40mM Tri-HCl 7.4; 25mM NaCl; 5mM MgCl2; 10% glycerol; 1mM pyruvate; 1mM ATP; 1mg / ml BSA) and incubated at 37 °C for 3h;
(2) The culture from the above step (1) was added 1ml Incubation buffer 2, washed three times, and centrifuged at 1000g/min under 4°C for 10min;
(3) The supernatant was removed to the greatest extent possible with a pipette, then the remaining material was re-suspended with 10µl Roche DNase I, 5µl 10×DNase I buffer (110mM Tris-HCl 7.4; 32.5mM MgCl2) and 35µl Incubation buffer 2 and incubated under in 37°C for 30min;
(4) The culture from the above step (3) was washed three times with 1ml washing buffer (10% glycerol; 10mM Tri-HCl 7.4; 150mM NaCl; 1mM EDTA) and centrifuged at 1000g/min under 4°C for 10min.

2. RNA extraction of the synthetic mitochondria using a Qiagen RNA Extraction Kit:
(1) The material from the centrifugation was re-suspended with 700µl QIAzol Lysis Reagent, mixed evenly using a pipette, and stood at room temperature for 5min;
(2) The suspension was combined with 140µl chloroform, well shaken for 15s, and stood at room temperature for 2 ∼ 3min;
(3) The mixture was centrifuged at 12000 g under 4°C for 15min;
(4) The supernatant was transferred to a new EP centrifuge tube (RNase free), combined with 1.5x absolute ethanol, and mixed by pipetting;
(5) The mixed solution was added to the spin column (column volume: 700µl; the remnant can be added in several times), and centrifuged at greater than 8000 g at room temperature for 15s;
(6) 700µl Buffer RWT was added before centrifuging at greater than 8000 g at room temperature for 15s;
(7) 500µl Buffer RPE was added before centrifuging at greater than 8000 g at room temperature for 15s;
(8) 500µl Buffer RPE was added before centrifuging at greater than 8000 g at room temperature for 2min;
(9) A new 2ml collection tube was used before centrifuging at 21000g / min at room temperature for 1min;
(10) The spin column was transferred to a new 1.5mlEP tube, combined with 40µl of RNase-free water, and centrifuged at greater than 8000 g at room temperature for 1min;
(11) The eluted RNA in the EP tube was re-applied to the column and centrifuged at greater than 8000 g at room temperature for 1min.

3. *In vitro* reverse transcription of the synthetic mitochondrial RNA:
(1) Digestion of DNA in the RNA: conducted for 30min with Promega DNase Iunder 37 °C, with digestion system as follows:

| | |
|---|---|
| 10X DNase I buffer | 1µl |
| DNase I | 1µl |
| RNA | 8µl |

(2) 1µl stop buffer was added after digestion and the treatment lasted for 10min under 65 °C;
(3) Reverse transcription of RNA: Digested RNA was treated for 5min under 65 °C, with the treatment system as follows:

| | |
|---|---|
| Digested RNA | 8µl |
| Random primer | 1 µl |
| 10mM dNTP | 1 µl |

Treated RNA was cooled on ice immediately after the treatment.
(4) The following reagents were added and mixed well:

| | |
|---|---|
| 10X RT buffer | 2µl |
| 25mM MgCl2 | 4µl |
| 0.1M DTT | 2µl |
| RNase OUT | 1µl |
| Super Script | 1µl |

The following temperature treatments were done sequentially:

| | |
|---|---|
| 25 °C | 10min |
| 50 °C | 50min |
| 85 °C | 5min |

Treated RNA was cooled on ice immediately after the treatment.
4. Identification of the transcript in the cDNA using PrimeSTAR enzyme:
(1) The PCR reaction system is as follows:

| | |
|---|---|
| 5×PrimeSTAR® Buffer (Mg²⁺ plus) | 10 µl |
| dNTP Mixture ( 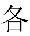 2.5 mM) | 4 µl |
| Primer 1 (10 µM) | 1 µl |
| Primer 2 (10 µM) | 1 µl |
| Template DNA | 2 µl |
| PrimeSTAR enzyme (2.5 U/µl) | 0.5 µl |
| Sterilized distilled water | 31.5 µl |

Reaction conditions:

| | |
|---|---|
| 98 °C | 10 sec |
| 55 °C | 12 sec |
| 72 °C | 1 min |

Number of cycles: 30.
(2) The PCR product was subjected to 1.5% agarose gel electrophoresis, and photographs were taken for detection. The obtained results shown in FIG. 2A, wherein the "positive" lane represents the PCR product obtained using, as a template, the cDNA that was the reverse-transcript of the isolated RNA from the NIH3T3 cells. The "assembly "lane represents the PCR product obtained using, as a template, the cDNA that was electrically isolated after electroporation. The" negative" lane represents the lane with the PCR product obtained using, only water as a template.

### V. In vitro replication and detection of the synthetic mitochondrial DNA:

1. The turned mitochondria precipitate was re-suspended with 50µl Incubation buffer 2, combined with dNTP containing 1 µCi alpha-32p-dCTP, and incubated at 37°C for 3h;
2. Total cellular DNA was collected using a Total DNA extraction kit (Tiangen);
3. Digestion was performed with BstUI restriction enzyme overnight at 65°C;
4 The digestion product was subjected to 5% acrylamide gel electrophoresis. The gel was removed afterwards, dried in the hood, detected with autoradiography exposure, the results of which shown in Fig. 2B. The "positive" lane represents the product obtained from the mitochondrial DNA from the mitochondrian isolated from the NIH3T3 cells after isotope incorporation. The "assembly "lane represents the product obtained from the mitochondrial DNA after electroporation after isotope incorporation. The" negative" lane represents the product obtained from only water after isotope incorporation.

### Example 4

In this embodiment, the process of incorporating the synthetic mitochondria containing GFP-COX-I fusion gene into mouse macrophage cell line by endocytosis is described, with the results shown in Figure 3.
(1) Mouse macrophage cell line RAW264.7 cell culture medium (high glucose DMEM (commercially available from Hyclone, Item No: SH30022.01B); 10% fetal bovine serum (commercially available from GIBCO, Item No: 16000-044); double antibody (including 10000U / ml penicillin and 10000µg / ml streptomycin, 1: 100 dilution for use, available from GIBCO, Item No: 15140-122)). Cells were cultured in culture dishes according to conventional methods.
(2) The precipitated synthetic mitochondria obtained after electroporation as illustrated in Example 3 were re-suspended with 200µL RAW264.7 cell culture medium. Aliquots of the suspension were added to culture dishes and incubated under 37°C and 5% CO₂ in a water-saturated closed incubator. GFP-expressing mouse macrophage cells containing the exogenous mitochondria could be observed in 12 hours after endocytosis. The culture was further incubated for 12 hours and observations were made for detection, the results shown in Fig. 3.

The results clearly indicate that our design of GFP-COX I fusion gene is able to properly express in mitochondria. In Fig. 3, Fig. 3A shows the staining results with a mitochondria-specific dye, Mitotracker Red. Fig. 3B is the GFP detection results. Fig. 3C is the DAPI staining results, indicating the position of the nucleus. Fig. 3D is the three-color overlay.

In this embodiment, it is confirmed that the designed GFP-COX I fusion gene can be stably expressed in macrophages. Of course, mitochondria obtained through isolation that are not genetically altered (e.g., isolated or synthetic mitochondrion, the DNA sequences of which are wild-type mammalian mitochondrial DNA) can also be stably expressed in macrophages after entering macrophages by endocytosis.

The detailed embodiments described herein are only for the purpose of illustrating the present invention, and are not intended to limit the scope of the present invention in any way. It would be understand by a person skilled in the art that various changes and modifications can be made to the embodiments described herein without departing from the scope and spirit of the present invention. Such changes and modifications are contemplated by the present invention, the scope of which should only be defined by the following claims.

## Claims

1. A macrophage comprising a synthetic mitochondrion, the synthetic mitochondrion is obtained by introducing a synthetic mitochondrial DNA into a mitochondrion or an empty mitochondrial shell, the synthetic mitochondrial DNA is circular mitochondrial DNA containing a GFP-COX-I fusion gene, and is stably expressed and stably passaged in the macrophage after entering the macrophage.

2. The macrophage according to claim 1, wherein the circular mitochondrial DNA is derived from inserting GFP gene and Linker sequences set forth in SEQ ID No.1 in the position 5328 of the mitochondrial DNA of wild type C57 BL / 6J mice.

3. The macrophage according to claim 1, wherein the empty mitochondrial shell is an empty mitochondrial shell of NIH3T3 RhoO cells.

4. The macrophage according to claim 1, wherein the macrophage is mouse macrophage cell line RAW264.7.

5. Method for preparing a macrophage of Claim 1, **characterized in that** the method comprises:
adding the synthetic mitochondrion to a cell culture system comprising the macrophage and continuing culturing to obtain the macrophage comprising the synthetic mitochondrion.

6. The method according to Claim 5, **characterized in that**
the culturing conditions are 36-38 °C in temperature and 4.5-5.5% CO₂ in a water-saturated closed incubator.

7. Method for introducing synthetic mitochondria into a macrophage, **characterized in that** the method comprises:
obtaining a synthetic mitochondrion by introducing a synthetic mitochondrial DNA into a mitochondrion or an empty mitochondrial shell, the synthetic mitochondrial DNA is circular mitochondrial DNA containing a GFP-COX-I fusion gene; and
adding the synthetic mitochondrion to a cell culture system comprising a macrophage and continuing culturing to obtain the macrophage comprising the synthetic mitochondrion, the synthetic mitochondrial DNA is stably expressed and stably passaged in the macrophage after entering the macrophage.

8. The method according to Claim 7, wherein the circular mitochondrial DNA is derived from inserting GFP gene and Linker sequences set forth in SEQ ID No.1 in the position 5328 of the mitochondrial DNA of wild type C57 BL / 6J mice.

9. The method according to Claim 7, wherein the empty mitochondrial shell is an empty mitochondrial shell of NIH3T3 RhoO cells.

10. The method according to Claim 7, wherein the macrophage is mouse macrophage cell line RAW264.7.

11. The method according to Claim 7, **characterized in that** the culturing conditions are 36-38 °C in temperature and 4.5-5.5% CO₂ in a water-saturated closed incubator.

## Patentansprüche

1. Makrophage, umfassend ein synthetisches Mitochondrium, wobei das synthetische Mitochondrium erhalten wird, indem eine synthetische Mitochondrien-DNA in ein Mitochondrium oder eine leere Mitochondrienhülle eingeführt wird, wobei es sich bei der synthetischen Mitochondrien-DNA um eine ringförmige Mitochondrien-DNA handelt, die ein GFP-COX-I-Fusionsgen enthält, und die im Makrophagen stabil exprimiert und stabil passagiert wird, nachdem sie in den Makrophagen gelangt ist.

2. Makrophage nach Anspruch 1, wobei die ringförmige Mitochondrien-DNA hergestellt wird, indem ein GFP-Gen und Linkersequenzen gemäß den Angaben in SEQ ID Nr. 1 an Position 5328 der Mitochondrien-DNA von C57 BL / 6J Wildtyp-Mäusen inseriert werden.

3. Makrophage nach Anspruch 1, wobei es sich bei der leeren Mitochondrienhülle um eine leere Mitochondrienhülle von NIH3T3 RhoO Zellen handelt.

4. Makrophage nach Anspruch 1, wobei es sich beim Makrophagen um die Maus-Makrophagenzelllinie RAW264.7 handelt.

5. Verfahren zur Herstellung eines Makrophagen nach Anspruch 1, **dadurch gekennzeichnet, dass** das Verfahren Folgendes umfasst:
das Zugeben des synthetischen Mitochondriums zu einem den Makrophagen umfassenden Zellkultursystem und das Fortsetzen des Kultivierens, um den das synthetische Mitochondrium umfassenden Makrophagen zu erhalten.

6. Verfahren nach Anspruch 5, **dadurch gekennzeichnet, dass** es sich bei den Kulturbedingungen um eine Temperatur von 36-38 °C und 4,5-5,5 % CO₂ in einem geschlossenen Inkubator mit wassergesättigter Atmosphäre handelt.

7. Verfahren zur Einführung von synthetischen Mitochondrien in einen Makrophagen, **dadurch gekennzeichnet, dass** das Verfahren Folgendes umfasst:
den Erhalt eines synthetischen Mitochondriums durch die Einführung einer synthetischen Mitochondrien-DNA in ein Mitochondrium oder eine leere Mitochondrienhülle, wobei es sich bei der synthetischen Mitochondrien-DNA um eine ringförmige Mitochondrien-DNA handelt, die ein GFP-COX-I-Fusionsgen enthält; und
das Zugeben des synthetischen Mitochondriums zu einem einen Makrophagen umfassenden Zellkultursystem und das Fortsetzen des Kultivierens, um den das synthetische Mitochondrium umfassenden Makrophagen zu erhalten, wobei die synthetische Mitochondrien-DNA im Makrophagen stabil exprimiert und stabil passagiert wird, nachdem sie in den Makrophagen gelangt ist.

8. Verfahren nach Anspruch 7, wobei die ringförmige Mitochondrien-DNA hergestellt wird, indem ein GFP-Gen und Linkersequenzen gemäß den Angaben in SEQ ID Nr. 1 an Position 5328 der Mitochondrien-DNA von C57 BL / 6J Wildtyp-Mäusen inseriert werden.

9. Verfahren nach Anspruch 7, wobei es sich bei der leeren Mitochondrienhülle um eine leere Mitochondrienhülle von NIH3T3 RhoO Zellen handelt.

10. Verfahren nach Anspruch 7, wobei es sich beim Makrophagen um die Maus-Makrophagenzelllinie RAW264.7 handelt.

11. Verfahren nach Anspruch 7, **dadurch gekennzeichnet, dass** es sich bei den Kulturbedingungen um eine Temperatur von 36-38 °C und 4,5-5,5 % CO₂ in einem geschlossenen Inkubator mit wassergesättigter Atmosphäre handelt.

## Revendications

1. Macrophage comprenant une mitochondrie synthétique, la mitochondrie synthétique étant obtenue par introduction d'un ADN mitochondrial synthétique dans une mitochondrie ou une enveloppe mitochondriale vide, l'ADN mitochondrial synthétique étant un ADN mitochondrial circulaire contenant un gène de fusion GFP-COX-I, et étant exprimé de façon stable et passé de façon stable dans le macrophage après entrée dans le macrophage.

2. Macrophage selon la revendication 1, dans lequel l'ADN mitochondrial circulaire est dérivé de l'insertion du gène GFP et de séquences de lieur décrites dans SEQ ID N° 1 à la position 5328 de l'ADN mitochondrial de souris C57 BL / 6J de type sauvage.

3. Macrophage selon la revendication 1, dans lequel l'enveloppe mitochondriale vide est une enveloppe mitochondriale vide de cellules NIH3T3 RhoO.

4. Macrophage selon la revendication 1, le macrophage étant la lignée cellulaire de macrophage de souris RAW264.7.

5. Procédé de préparation d'un macrophage selon la revendication 1, **caractérisé en ce que** le procédé comprend :
l'ajout de la mitochondrie synthétique à un système de culture de cellules comprenant le macrophage et la poursuite de la culture pour obtenir le macrophage comprenant la mitochondrie synthétique.

6. Procédé selon la revendication 5, **caractérisé en ce que**
les conditions de culture sont une température de 36 à 38 °C et 4,5 à 5,5 % de CO₂ dans un incubateur fermé saturé en eau.

7. Procédé d'introduction de mitochondries synthétiques dans un macrophage, **caractérisé en ce que** le procédé comprend :
l'obtention d'une mitochondrie synthétique par introduction d'un ADN mitochondrial synthétique dans une mitochondrie ou une enveloppe mitochondriale vide, l'ADN mitochondrial synthétique étant un ADN mitochondrial circulaire contenant un gène de fusion GFP-COX-I ; et
l'ajout de la mitochondrie synthétique à un système de culture de cellules comprenant un macrophage et la poursuite de la culture pour obtenir le macrophage comprenant la mitochondrie synthétique, l'ADN mitochondrial synthétique est exprimé de façon stable et passé de façon stable dans le macrophage après entrée dans le macrophage.

8. Procédé selon la revendication 7, dans lequel l'ADN mitochondrial circulaire est dérivé de l'insertion du gène GFP et de séquences de lieur décrites dans SEQ ID N° 1 à la position 5328 de l'ADN mitochondrial de souris C57 BL / 6J de type sauvage.

9. Procédé selon la revendication 7, dans lequel l'enveloppe mitochondriale vide est une enveloppe mitochondriale vide de cellules NIH3T3 RhoO.

10. Procédé selon la revendication 7, dans lequel le macrophage est la lignée cellulaire de macrophage de souris RAW264.7.

11. Procédé selon la revendication 7, **caractérisé en ce que** les conditions de culture sont une température de 36 à 38 °C et 4,5 à 5,5 % de CO₂ dans un incubateur fermé saturé en eau.
